# EUROPÄISCHE PATENTSCHRIFT

(11) **EP 2 624 793 B1**
(45) Veröffentlichungstag und Bekanntmachung des Hinweises auf die Patenterteilung: **24.06.2015**
(21) Anmeldenummer: 11760800.0
(22) Anmeldetag: 27.09.2011
(51) Int. Cl.: A61F 9/00, A61M 5/20

(54) **AUSTRAGVORRICHTUNG**
DISCHARGE DEVICE
DISPOSITIF DE DISTRIBUTION

(30) Priorität: 04.10.2010 DE 102010048085
(43) Veröffentlichungstag der Anmeldung: 14.08.2013
(73) Patentinhaber: Aptar Radolfzell GmbH, 78315 Radolfzell (DE)
(72) Erfinder: GREINER-PERTH, Jürgen, 78244 Gottmadigen (DE); WOCHELE, Matthias, 78239 Rielasingen-Worblingen (DE)
(74) Vertreter: Patentanwälte Ruff, Wilhelm, Beier, Dauster & Partner
(86) Internationale Anmeldenummer: PCT/EP2011/066778
(87) Internationale Veröffentlichungsnummer: WO 2012/045614

(56) Entgegenhaltungen:
- EP-A1- 0 378 935
- US-A- 5 085 642
- US-A1- 2004 015 126

## Beschreibung

Die Erfindung betrifft eine Austragvorrichtung zum Austrag pharmazeutischer Flüssigkeiten, insbesondere zu ophthalmischen Zwecken, mit einem Gehäuse, einem Flüssigkeitsspeicher mit einem Aufnahmeraum zur Speicherung der pharmazeutischen Flüssigkeit vor dem Austrag und einer am Gehäuse vorgesehenen Austragöffnung, die den Aufnahmeraum mit einer Umgebung verbindet und der Abgabe der pharmazeutischen Flüssigkeit dient.

Solche Austragvorrichtungen sind aus dem Stand der Technik vielfach bekannt. Bei solchen gattungsgemäßen Austragvorrichtungen bedarf es eines Überdrucks, um die pharmazeutische Flüssigkeit vom Aufnahmeraum zur Austragvorrichtung zu fördern. Die Betätigung einer Vielzahl bekannter Austragvorrichtungen erfolgt derart, dass dieser Druck manuell erzeugt wird, beispielsweise durch Betätigung einer als Fördereinrichtung dienenden Pumpe oder durch Zusammendrücken des Flüssigkeitsspeichers selbst und eine dadurch erzielte Druckerhöhung im Aufnahmeraum.

Bei vielen bekannten Austragvorrichtungen wird dieser zum Flüssigkeitsaustrag erzeugte Druck auch genutzt, um hierdurch ein druckgesteuertes Auslassventil zu öffnen, welches im Ruhezustand die Austragöffnung der Austragvorrichtung verschließt.

Insbesondere bei Austragvorrichtungen für ophthalmische Zwecke wird jedoch ein geringer Flüssigkeitsdruck an der Austragöffnung gewünscht, so dass ein dort vorgesehenes druckgesteuertes Auslassventil schwer zu realisieren ist, da es durch eine nur geringe Differenz zwischen zwei Drücken zwischen einem zuverlässig schließenden Zustand und einem zuverlässig öffnenden Zustand umschalten können muss.

Aus der US 2004/015126 A1 ist eine Austragvorrichtung mit einer Federeinrichtung bekannt, welche zum Zwecke des Austrags gespannt wird, beispielsweise durch ein Verdrehen zweier Gehäuseteile. Nach dem Spannen kann die Federeinrichtung mittels einer Auslösevorrichtung ausgelöst werden.

### Aufgabe und Lösung

Es ist daher primär Aufgabe der vorliegenden Erfindung, eine gattungsgemäße Austragvorrichtung dahingehend weiterzubilden, dass diese verbesserte Austragvorrichtungen, insbesondere zum Austrag von Flüssigkeit in Tropfenform, zur Verfügung zu stellen.

Erfindungsgemäß wird dies dadurch erreicht, dass eine vorgespannte Federeinrichtung an einer erfindungsgemäßen Austragvorrichtung vorgesehen ist, mittels derer Flüssigkeit im Aufnahmeraum des Flüssigkeitsspeichers druckbeaufschlagt wird und die im montierten Zustand der Austragvorrichtung nicht zur Einbringung von Energie zugänglich ist.

Bei einer erfindungsgemäßen Austragvorrichtung wird somit mittels einer Federeinrichtung, in der mechanische Arbeit gespeichert ist, Druck auf die Flüssigkeit im Flüssigkeitsspeicher ausgeübt. Da die Flüssigkeit somit zumindest ab dem Zeitpunkt der Erstinbetriebnahme druckbeaufschlagt ist, muss zuverlässig gewährleistet sein, dass diese Druckbeaufschlagung nicht für sich alleine genommen einen Flüssigkeitsaustritt durch die Austragöffnung gewährleistet. Maßnahmen hierfür werden im Weiteren noch erläutert.

Die Federeinrichtung einer erfindungsgemäßen Austragvorrichtung ist erfindungsgemäß im montierten Zustand der Austragvorrichtung nicht zur Einbringung von Energie zugänglich. Somit ist in einem Auslieferzustand der Austragvorrichtung, in der die verschiedenen Teilbestandteile der Austragvorrichtung bereits zusammengefügt wurden, kein weiteres Spannen der Federeinrichtung möglich. Stattdessen wird die Federeinrichtung bereits vor Auslieferung vollständig gespannt und diese in der Feder gespeicherte Energie wird während der Nutzung Schritt für Schritt zur Ausbringung der Flüssigkeit genutzt.

Die in einer im Bereich der Austragvorrichtungen üblichen Feder speicherbare mechanische Arbeit ist nicht sehr groß. Dies ist jedoch insbesondere im Hinblick auf den bevorzugten Anwendungszweck der Tropfenspender von nachrangiger Bedeutung, da zur Ausbringung der Flüssigkeit mit einem Überdruck zwischen 100 und 300 mbar, wie bei Tropfern üblich, bereits sehr geringe Energiemengen ausreichen.

Besonders bevorzugt ist es, wenn bei einer erfindungsgemäßen Austragvorrichtung ein manuell mittels einer Betätigungshandhabe betätigbares Auslassventil vorgesehen ist, welches zwischen dem Aufnahmeraum und der Austragöffnung angeordnet ist. Ein solches manuell betätigbares Auslassventil kann im geschlossenen Zustand mittels vergleichsweise starker Federn geschlossen gehalten werden, da die Überwindung einer Federkraft einer solchen sehr starken Feder (beispielsweise zwischen 3 und 10 Newton) bei einer manuellen Betätigung ohne Weiteres überwunden werden kann. Flüssigkeit, die nur unter geringem Überdruck steht, vermag eine solche Federkraft jedoch nur bei sehr großen Druckbeaufschlagungsflächen zu überwinden, die in handliche und tragbare Austragvorrichtungen nur schwer integrierbar sind.

Bei einer Austragvorrichtung gemäß der genannten Weiterbildung steht das Medium somit permanent und bis zum Auslassventil unter dem von der Federeinrichtung erzeugten Druck. Sobald eine manuelle Betätigung des Auslassventils erfolgt und dieses geöffnet wird, tritt die Flüssigkeit unter dem vorzugsweise geringen Überdruck durch die Austragöffnung durch und bildet dort vorzugsweise Tropfen zur ophthalmischen Applizierung.

Bei einer Weiterbildung der Erfindung ist vorgesehen, dass die Austragvorrichtung zur Abgabe der Flüssigkeit in Tropfenform ausgebildet ist. Hierzu ist es vorzugsweise insbesondere vorgesehen, dass bei bestimmungsgemäßer Betätigung die Flüssigkeit mit einem nur sehr geringen Überdruck von vorzugsweise weniger als 500 mbar, insbesondere vorzugsweise bei einem Überdruck zwischen 100 mbar und 300 mbar, durch die Austragöffnung nach außen strömt. Wie oben bereits erläutert, kann ein solch geringer Überdruck direkt durch die Federeinrichtung zur Druckbeaufschlagung des Flüssigkeitsspeichers erzielt werden. Allerdings ist es auch möglich, die Federeinrichtung stärker und den im Aufnahmeraum dadurch entstehenden Druck höher auszulegen, wobei in einem solchen Falle vorzugsweise eine Drosseleinrichtung zwischen dem Flüssigkeitsspeicher und dem Auslassventil vorgesehen ist, welcher eine Druckreduktion auf den oben genannten bevorzugten Druckbereich verursacht.

Als Federeinrichtung zur Druckbeaufschlagung des Flüssigkeitsspeichers kommen verschiedene Formen von Federn in Frage, beispielsweise auch Gasdruckfedern. Die Druckbeaufschlagung kann durch Komprimierung eines in sich verformbaren Flüssigkeitsspeichers, wie beispielsweise eines Beutels oder eines Balgs, erfolgen.

Bevorzugt ist allerdings, wenn die Federeinrichtung durch eine Flüssigkeitsspeicherfeder in Form einer elastisch deformierbaren Feder gebildet wird, mittels derer die Flüssigkeit im Aufnahmeraum kraftbeaufschlagt wird, wobei die Flüssigkeitsspeicherfeder insbesondere auf einen den Aufnahmeraum in einer Richtung begrenzenden und im Flüssigkeitsspeicher gleitbeweglichen Kolben wirkt.

Eine solche Flüssigkeitsspeicherfeder kann beispielsweise als Schraubenfeder ausgebildet sein. Eine besonders naheliegende Ausgestaltung sieht die Verwendung einer metallischen Feder hierfür vor. Es sind jedoch auch andere Federn, insbesondere beispielsweise Kunststofffedern, für diesen Zweck geeignet, wobei eine solche Kunststofffeder insbesondere einstückiger Teil des Kolbens sein kann und somit eine sehr einfache und kostengünstige Bauweise gestattet.

Von besonderem Vorteil ist es, wenn der Kolben den Aufnahmeraum auf der der Austragöffnung zugewandten Seite begrenzt und die Federeinrichtung derart ausgebildet und/oder angeordnet ist, dass sie den Kolben von der Austragöffnung weg kraftbeaufschlagt. Bei einer solchen Gestaltung erfolgt die Kraftbeaufschlagung der Flüssigkeit durch den Kolben demzufolge in entgegengesetzter Richtung zur Fließrichtung der Flüssigkeit vom Aufnahmeraum des Flüssigkeitsspeichers zur Austragöffnung. Hierdurch ist insbesondere eine Bauweise realisierbar, derzufolge der Flüssigkeitsspeicher als vom Gehäuse getrenntes und mit diesem verbindbares Bauteil ausgebildet ist, wobei der Kolben bereits vor der Verbindung des Flüssigkeitsspeichers mit dem Gehäuse am Gehäuse beweglich gesichert ist. Der Flüssigkeitsspeicher kann bei einer solchen Gestaltung sehr einfach ausgebildet sein, insbesondere als einstückiges, napfartiges Bauteil, welches bei der Befestigung am Gehäuse der Austragvorrichtung gleichzeitig den an der Baugruppe des Gehäuses vorgesehenen Kolben aufnimmt. Dies ist insbesondere im Hinblick auf ein nachfolgend noch erläutertes Verfahren zur Einbringung der Federenergie in die Federeinrichtung von Vorteil.

Bei der Gestaltung, bei der die Bewegungsrichtung des Kolbens der Bewegungsrichtung der Flüssigkeit vom Aufnahmeraum zur Austragöffnung entgegenläuft, wird es als besonders bevorzugt angesehen, wenn der Kolben auf einem Hohlrohr gleitbeweglich geführt ist, welches den Aufnahmeraum des Flüssigkeitsspeichers mit der Austragöffnung verbindet. Dieses Hohlrohr übernimmt dabei somit zwei Funktionen. Zum einen leitet es die Flüssigkeit am Kolben vorbei in Richtung der Austragöffnung. Zum anderen stellt es eine Führung für den Kolben dar, insbesondere für einen Zeitraum vor der Montage der Austragvorrichtung, in der der Kolben noch nicht innerhalb des Flüssigkeitsspeichers geführt ist.

Besonders bevorzugt ist es, wenn das Hohlrohr mit einem Ventilkörper des Auslassventils einstückig ausgebildet oder fest verbunden ist. Durch eine solche Ausgestaltung ist eine Gestaltung der Austragvorrichtung mit sehr wenigen Bauteilen möglich, da keine zwei Bauteile für den Ventilkörper und das Hohlrohr gebraucht werden.

Bei einer besonders bevorzugten Ausgestaltung, bei der der Kolben beweglich am Gehäuse gesichert ist, ist weiterhin vorgesehen, dass das Gehäuse und die Relativbeweglichkeit des Kolbens gegenüber dem Gehäuse derart aufeinander abgestimmt sind, dass der Kolben nicht oder nur in geringem Maße (kleiner 10 mm) über das der Austragöffnung abgewandte Ende des Gehäuses hinaus gegenüber diesem verlagerbar ist. Vorzugsweise wird dies dadurch erreicht, dass das Gehäuse auf der Austragöffnung abgewandten Seite eine umlaufende Schürze oder anderweitige Schutzstege aufweist, die somit einen Schutz für den Kolben vor dessen Einfügung in den Flüssigkeitsspeicher im Zuge der Montage darstellen. Dieser Schutz, der durch das Gehäuse und die begrenzte Relativbeweglichkeit des Kolbens erzielt wird, ist insbesondere von Vorteil, wenn die Komponenten der Austragvorrichtung vor der Montage als Schüttgut gehandhabt werden, so dass ohne einen Schutz für den Kolben eine Verletzung des Kolbens und somit eine spätere Undichtigkeit entstehen könnten.

Bei einer besonders bevorzugten Ausgestaltung ist ein Ventilkörper am Auslassventil vorgesehen, wobei dieser Ventilkörper mittels einer Ventilfeder in Richtung eines Schließzustandes kraftbeaufschlagt ist. Dabei ist die Ventilfeder vorzugsweise mit der Federeinrichtung zur Druckbeaufschlagung der Flüssigkeit, insbesondere mit der genannten Flüssigkeitsspeicherfeder, identisch. Eine Ventilfeder zum Kraftbeaufschlagen des Ventilkörpers in Richtung seines Schließzustandes ist auch bei einer Gestaltung der Austragvorrichtung von Vorteil, bei der die Öffnung des Auslassventils mittels einer manuellen Handhabe unmittelbar mechanisch erzielt wird, da hierdurch zuverlässig nach Loslassen der Handhabe der Schließzustand wieder hergestellt wird. Durch die Verwendung einer gemeinsamen Feder zur Druckbeaufschlagung der Flüssigkeit im Aufnahmeraum und zum Schließen des Auslassventils kann eine erfindungsgemäße Austragvorrichtung mit einer sehr geringen Zahl an Pfeilen ausgebildet sein.

Da jedoch eine Feder zum Schließen des Auslassventils insbesondere bei einem Auslassventil, welches manuell mittels einer Handhabe geöffnet wird, vergleichsweise stark ausgebildet sein kann und daher auch der Flüssigkeitsdruck im Aufnahmeraum, der durch diese Feder erzeugt wird, vergleichsweise hoch sein kann, kann es insbesondere bei einer solchen Gestaltung mit nur einer Feder für das Auslassventil und für die Druckbeaufschlagung der Flüssigkeit von Vorteil sein, eine oben beschriebene Drosseleinrichtung vorzusehen.

Grundsätzlich ist es möglich, bei einer erfindungsgemäßen Austragvorrichtung bereits ab dem Zeitpunkt der Montage die Druckbeaufschlagung der Flüssigkeit durch die im Zuge der Montage gespannte Federeinrichtung vorzusehen. Ein höheres Maß an Sicherheit gegen ungewollten Austrag von Flüssigkeit bereits vor der Inbetriebnahme beim Endkunden kann jedoch erreicht werden, wenn ein von außen zugängliches und bei der Inbetriebnahme handhabbares Sicherungsmittel vorgesehen ist, mittels dessen die Federeinrichtung vor Inbetriebnahme im vorgespannten Zustand gehalten wird. Dieses Sicherungsmittel kann im Zuge der Inbetriebnahme durch den Endkunden gelöst werden, so dass sich erst zu diesem Zeitpunkt die gewünschte Druckbeaufschlagung der Flüssigkeit im Aufnahmeraum einstellt.

Die Erfindung betrifft weiterhin ein Montageverfahren für eine Austragvorrichtung für pharmazeutische Flüssigkeiten, wobei die Austragvorrichtung eine erste Baugruppe mit einem Gehäuse mit einer Austrittsöffnung und einem manuell betätigbaren Auslassventil, eine zweite Baugruppe mit einem Flüssigkeitsspeicher mit einem Aufnahmeraum zur Aufnahme der Flüssigkeit und eine Federeinrichtung, die im gespannten Zustand nach der Montage die Flüssigkeit im Aufnahmeraum unter Druck setzt, umfasst. Dabei wird erfindungsgemäß ausgehend von einem Ausgangszustand, in dem das Gehäuse und der Flüssigkeitsspeicher als getrennte Baugruppen vorliegen, folgende Schrittfolge durchgeführt: Zunächst wird der Aufnahmeraum des Flüssigkeitsspeichers mit der pharmazeutischen Flüssigkeit befüllt. Anschließend werden die beiden Baugruppen miteinander verbunden, wobei durch das Verbinden der Baugruppen die Federeinrichtung gespannt wird und somit der Druck zur Druckbeaufschlagung der Flüssigkeit im Aufnahmeraum aufgebaut wird.

Bei dem erfindungsgemäßen Montageverfahren wird somit durch das Zusammenführen der beiden Baugruppen jene Energie eingebracht, die anschließend in der Federeinrichtung gespeichert wird, bis sie im Zuge eines Austragvorgangs bzw. im Zuge des Befüllens einer Pumpkammer genutzt wird. Während des Zusammenfügens der Baugruppen wird also gleichzeitig die Federeinrichtung gespannt. Anschließend kann zur Verringerung der Druckbeaufschlagung der Flüssigkeit während der Lagerung ein Sicherungsstift oder dergleichen eingesetzt werden, um die Feder im gespannten Zustand zu halten. Es bedarf bei dieser Form der Montage keines separaten Schrittes zum Spannen der Federeinrichtung.

Besonders von Vorteil ist es, wenn die erste Baugruppe einen Kolben und die genannte Federeinrichtung aufweist, wobei nach Befüllen des Aufnahmeraums im ersten Schritt der Aufnahme zunächst endseitig offen bleibt und erst im darauffolgenden Schritt durch das Einrücken des Kolbens in den Flüssigkeitsspeicher gegenüber der Umgebung verschlossen wird. Eine fortgeführte Bewegung der Baugruppen aufeinander zu zum Verbinden der Baugruppen führt dann, nach Verschließen des Aufnahmeraums durch den Kolben, zum Spannen der Federeinrichtung.

### Kurzbeschreibung der Zeichnungen

Weitere Aspekte und Vorteile der vorliegenden Erfindung ergeben sich außer aus den Ansprüchen auch aus der nachfolgenden Beschreibung von bevorzugten Ausführungsbeispielen der Erfindung, die anhand der Figuren erläutert werden. Dabei zeigen in schematischer Weise:
- Fig. 1a und 1b: eine erste Ausführungsform einer erfindungsgemäßen Austragvorrichtung vor und während eines Austragvorgangs,
- Fig. 2a bis 2c: die Austragvorrichtung der Fig. 1a und 1b in verschiedenen Stadien während der Montage und
- Fig. 3 und 4: Varianten zur Ausführungsform der Fig. 1 und 2.

### Detaillierte Beschreibung der Ausführungsbeispiele

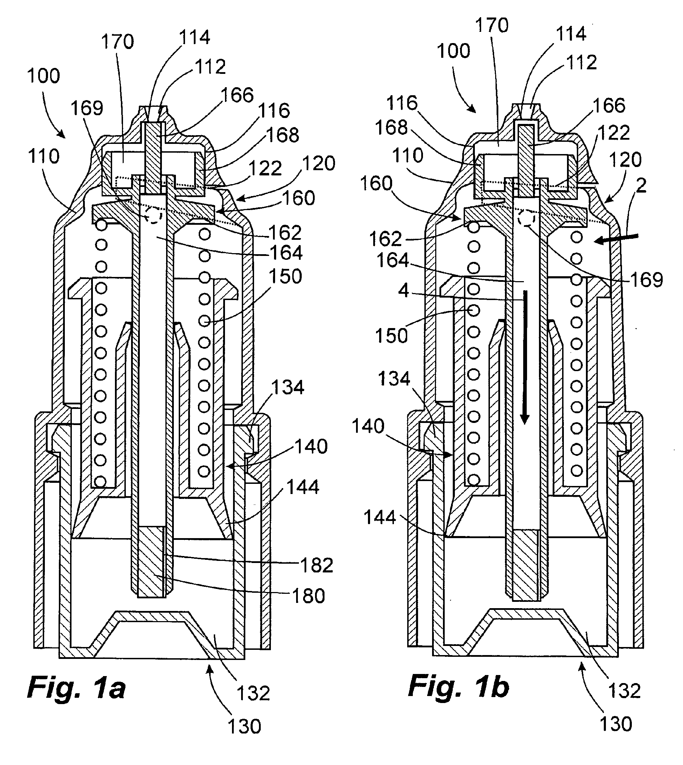

Die Fig. 1a und 1b zeigen eine erste Ausführungsform einer erfindungsgemäßen Austragvorrichtung 100.

Bezug nehmend auf die Darstellung der Fig. 1a werden zunächst die Einzelbauteile dieser Austragvorrichtung erläutert. Die Austragvorrichtung 100 weist ein Gehäuse 110 auf, welches im Wesentlichen als Rotationskörper ausgebildet ist und an einer Stirnseite eine Austragöffnung 112 aufweist. Am gegenüberliegenden Ende ist das Gehäuse 110 offen ausgebildet und wird durch einen Flüssigkeitsspeicher 130 abgeschlossen, der einen Aufnahmeraum 132 zur Aufnahme einer auszutragenden Flüssigkeit aufweist. Der Flüssigkeitsspeicher 130 ist durch eine Rastverbindung 134 in das Gehäuse 110 eingerastet und damit im Betrieb zu diesem ortsfest.

Zur Volumenreduktion des Aufnahmeraums 132 ist dieser nach oben hin durch einen Kolben 140 abgeschlossen. Dieser Kolben 140, der den Aufnahmeraum 132 mittels einer Kolbenlippe 144 begrenzt, wird durch eine Feder 150 permanent nach unten kraftbeaufschlagt, um einen Druck auf die Flüssigkeit im Aufnahmeraum 132 auszuüben. Das dem Kolben 140 gegenüberliegende Ende der als Schraubenfeder ausgebildeten Feder 150 stützt sich an einer Anlagefläche 162 ab, die Teil eines Bauteils 160 ist, welches mehrere Funktionen übernimmt. Neben der zur Verfügungstellung der Stützfläche 162 für die Feder 150 bildet das Bauteil 160 auch einen Hohlkanal 164, der sich vom Aufnahmeraum 132 bis in den Bereich der Austragöffnung 112 erstreckt. Oberhalb der Stützfläche 162 weist das Bauteil 160 einen Ventilkörper 166 auf, der durch die Feder 150 gegen einen Ventilsitz 114 an der Innenseite des Gehäuses 110 gedrückt wird. Flüssigkeit, die im Betrieb am oberen Ende des Hohlrohrs 164 aus diesem austritt, gelangt in einen Ventilvorraum 170, der außenseitig durch einen Gleitkragen 168 des Bauteils 160 begrenzt wird, welcher seinerseits weitgehend flüssigkeitsdicht in einem Führungsabschnitt 116 an der Innenseite des Gehäuses 110 geführt ist.

Die Feder 150 übernimmt somit eine Doppelfunktion: Sie beaufschlagt über den Kolben 140 die Flüssigkeit im Aufnahmeraum 132 mit Druck. Darüber hinaus hält sie das Auslassventil 114, 164 in einem Ruhezustand der Austragvorrichtung 100 geschlossen.

Zur besseren Veranschaulichung ist in den Darstellungen der Fig. 1a und 1b die Flüssigkeit nicht mit eingezeichnet. Für die nachfolgende Erläuterung ist davon auszugehen, dass der Aufnahmeraum 132, das Hohlrohr 164 und die Ventilvorkammer 170 bereits vollständig mit Flüssigkeit befüllt sind.

In dem Zustand der Fig. 1a steht diese Flüssigkeit unter einem homogenen Druck, der durch die Feder 150 verursacht wird. Da die Feder eine vergleichsweise hohe Federkraft von 8 Newton zur Verfügung stellt, beträgt der Druck der Flüssigkeit vor Beginn des Austragvorgangs etwa 1 bar.

Zum Austrag von Flüssigkeit wird eine Betätigungshandhabe 120, die einstückiger Teil des Gehäuses 110 ist und mittels Freischnitten gegenüber dem übrigen Gehäuse 110 eindrückbar ist, in der in Fig. 1b mittels des Pfeils 2 dargestellten Weise eingedrückt, wobei durch einen gepunktet angedeuteten und an der Betätigungshandhabe 120 vorgesehenen Betätigungsarm 122 ein an der Baueinheit 160 vorgesehener Bolzen 169 niedergedrückt wird.

Gemeinsam mit dem Bolzen 169 wird die gesamte Baueinheit 160 in Richtung des Pfeils 4 verlagert, wobei dies ein Öffnen des Auslassventils 114, 164 gegen die Kraft der Feder 150 verursacht. Durch das Öffnen des Auslassventils 114, 164 bricht der Druck im Hohlrohr 164 und der Ventilvorkammer 170 augenblicklich zusammen, da aufgrund eines am unteren Ende in das Hohlrohr 164 eingefügten Drosselkörpers 180 mit einem engen Drosselkanal 182 mit einem freien Querschnitt von weniger als 1 mm² das Nachströmen von Flüssigkeit aus dem Aufnahmeraum 132 in das Hohlrohr 164 und die Ventilvorkammer 170 stark limitiert ist. Der Überdruck von 1 bar im Aufnahmeraum 132 wird durch den Drosselkanal 182 auf einen Überdruck von etwa 250 mbar im Hohlrohr 164 und der Ventilvorkammer 170 reduziert, so dass die Flüssigkeit durch die Austragöffnung 112 mit sehr geringem Druck hindurchströmt und somit zur Tropfenbildung an der Austragöffnung 112 geeignet ist.

Die dargestellte und beschriebene Austragvorrichtung 100 bildet eine aus nur wenigen einfach herzustellenden Bauteilen gebildete Austragvorrichtung, die eine sichere Handhabung und einen gut reproduzierbaren Flüssigkeitsaustrag gewährleistet.

Darüber hinaus ist die beschriebene Austragvorrichtung 100 für ein vorteilhaftes Montageverfahren geeignet, welches nachfolgend erläutert wird.

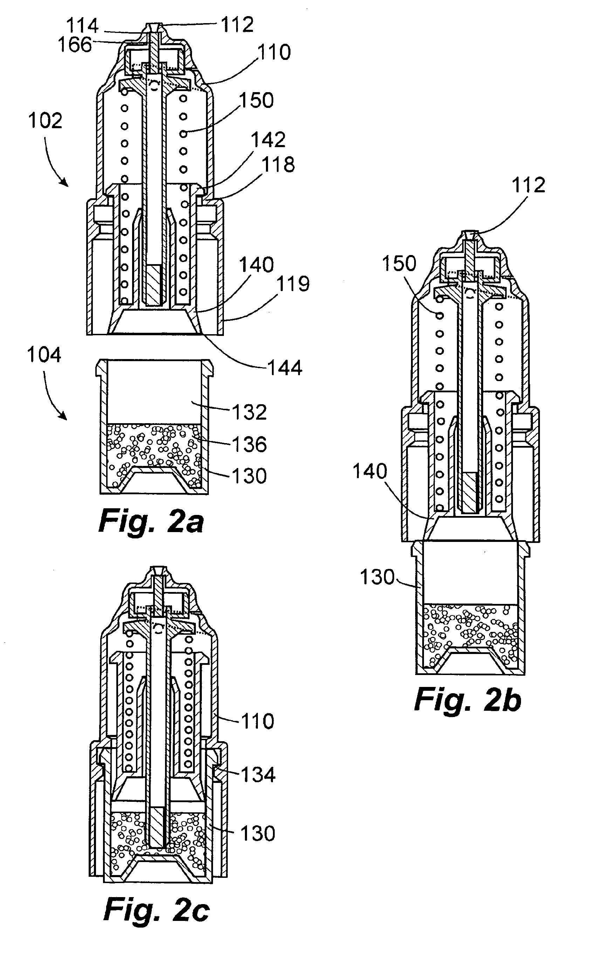

Die Montage der Austragvorrichtung der Fig. 1 a und 1 b wird anhand der Fig. 2a bis 2c erläutert.

Ausgangspunkt der Montage sind zwei getrennte Baugruppen 102, 104, wobei die Baugruppe 102 alle Bauteile der Austragvorrichtung 100 mit Ausnahme des Flüssigkeitsspeichers 130 umfasst. Insbesondere ist auch der Kolben 140 Teil der Baugruppe 102, wobei durch einen nach innen ragenden Vorsprung 118 am Gehäuse 110 sowie einen nach außen ragenden Kragen 142 am Kolben 140 gewährleistet ist, dass der Kolben 140 trotz der Kraft der Feder 150 nicht vom Gehäuse 110 getrennt werden kann. Stattdessen wird der Kolben durch die Feder 150 vor der Zusammenfügung der ersten Baugruppe 102 mit der zweiten Baugruppe 104 in eine untere Endlage gedrückt, in der er durch die Ausgestaltung des Gehäuses 110 geschützt ist. Dies wird durch eine umlaufende Schürze 119 am unteren Ende des Gehäuses 120 erzielt, welche die Kolbenlippe 144 des Kolbens 140 nach unten hin überragt. Hierdurch ist der Kolben 140 und insbesondere die Kolbenlippe 144 auch für den Fall geschützt, dass eine Vielzahl von Baugruppen 102 vor der Montage gemeinsam als Schüttgut gehandhabt wird.

Zur Fertigstellung der Austragvorrichtung der Fig. 1 a und 1 b wird zunächst der Aufnahmeraum 132 des Flüssigkeitsspeichers 130 mit Flüssigkeit 136 gefüllt. Anschließend werden die beiden Baugruppen 102, 104 aneinander angenähert, bis der Kolben 140 mit der Kolbenlippe 144 in den Flüssigkeitsspeicher 130 eindringt, so dass die Kolbenlippe 144 an der Innenseite des Flüssigkeitsspeichers 130 umlaufend zum Anliegen kommt. Dieser Zustand ist in Fig. 1b dargestellt.

Ab diesem Zeitpunkt kann Flüssigkeit und Luft nur noch durch die Austragöffnung 112 entweichen, wobei diese während des Montageprozesses durch die Federkraft der Feder 150 geschlossen gehalten wird. Beim fortgesetzten Einrücken der Baugruppe 104 mit dem Flüssigkeitsspeicher 130 in die Baugruppe 102 dringt der Kolben 140 tiefer in den Flüssigkeitsspeicher 130 ein, wobei die Feder 150 gespannt wird und bereits zu diesem Zeitpunkt die Druckbeaufschlagung der Flüssigkeit 136 bewirkt.

Der Montagevorgang ist beendet, sobald in der in Fig. 2c dargestellten Weise die Rastverbindung 134 zwischen dem Gehäuse 110 und dem Flüssigkeitsspeicher 130 hergestellt wurde. Die Austragvorrichtung befindet sich nun in einem Auslieferungszustand. Durch die vergleichsweise starke Feder 150 ist gewährleistet, dass das Auslassventil 114, 166 geschlossen gehalten wird, bis durch einen Benutzer mittels der Handhabe 120 bewusst ein Austragvorgang verursacht wird.

Das beschriebene Verfahren gestattet somit, die Federeinrichtung 150, die später die Energiequelle zum Austrag von Flüssigkeit aus der Austragvorrichtung 100 darstellt, bereits während der Montage und ohne zusätzliche hierfür erforderliche Schritte einzubringen.

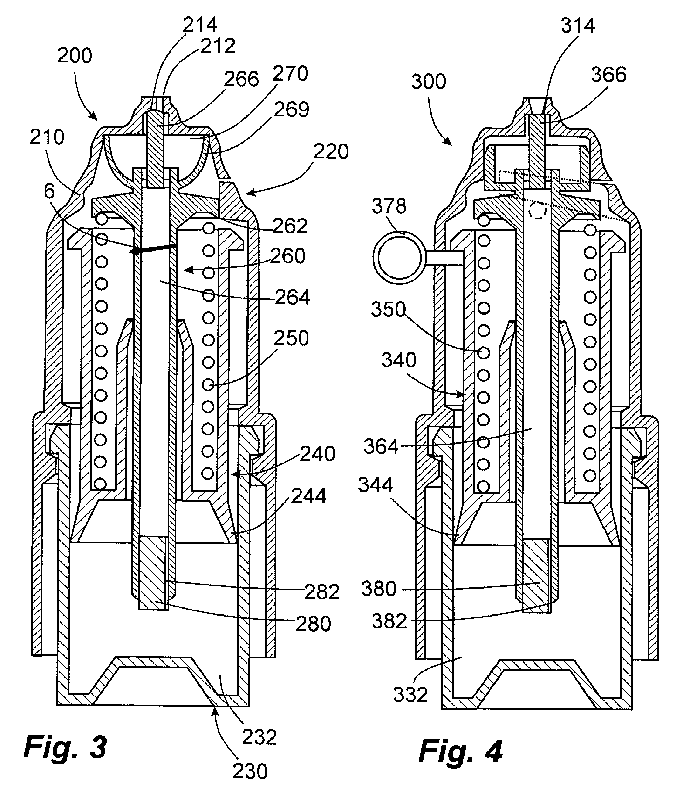

Die Fig. 3 und 4 zeigen Abwandlungen der Ausgestaltungen der Fig. 1 und 2, wobei funktionsähnliche oder funktionsidentische Bauteile hinsichtlich der beiden letzten Ziffern der Bezugsnummern mit denen der Ausführungsform der Fig. 1 und 2 übereinstimmen.

Bei der Ausführungsform der Fig. 3 liegt die Besonderheit in der Art der Betätigung. Statt die Baueinheit 260 in der Art der Ausführungsform der Fig. 1 und 2 in Richtung des Pfeils 4 mittels der Betätigungshandhabe 222 zu verlagern, wird die Baueinheit 260 in Richtung des Pfeils 6 mittels der Betätigungshandhabe 220 verkippt, wobei in Reaktion der Ventilkörper 266 gegenüber der Ventilfläche 214 leicht verschoben wird und hierdurch die Austragöffnung 212 freigibt. Sobald die Betätigungskraft auf die Betätigungshandhabe 222 entfällt, wird aufgrund der Formgebung der Ventilfläche 214 und unter der Wirkung der Feder 250 nach Wegfall der Betätigungskraft der Ventilkörper 264 wieder in die in Fig. 3 dargestellte Ausgangslage verschoben, in der er die Austragöffnung 212 versperrt. Diese Beweglichkeit des Bauteils 260 macht es erforderlich, statt des Gleitkragens 168 der Ausgestaltung der Fig. 1 und 2 einen formveränderlichen Kragen 268 vorzusehen, welcher die Ventilvorkammer 270 auch bei Verkippung des Bauteils 260 nach unten geschlossen hält.

Die Austragvorrichtung 300 gemäß der Fig. 4 ist nahezu identisch mit der Austragvorrichtung der Fig. 1 und 2. Einziger Unterschied ist, dass ein Sicherungsstift 378 vorgesehen ist, welcher im eingesetzten Zustand der Fig. 4 eine Verlagerung des Kolbens 340 unter dem Eindruck der Federkraft der Feder 350 in Richtung des Aufnahmeraums 332 verhindert und damit die Druckbeaufschlagung der Flüssigkeit im Aufnahmeraum 332 begrenzt. Der Sicherungsstift 378 wird bestimmungsgemäß vor Inbetriebnahme durch den Endbenutzer entfernt, so dass sich anschließend die gleiche Verhaltensweise der Austragvorrichtung 300 wie bei der Austragvorrichtung 100 der Fig. 1 und 2 einstellt. Bis zu diesem Zeitpunkt ist jedoch durch den Sicherungsstift der Flüssigkeitsdruck wirksam limitiert, so dass die Gefahr eines ungewollten Austretens von Flüssigkeit weiter verringert wird.

## Patentansprüche

1. Austragvorrichtung (100; 200; 300) zum Austrag pharmazeutischer Flüssigkeiten (136), insbesondere zu ophthalmischen Zwecken, mit
- einem Gehäuse (110; 210; 310),
- einem Flüssigkeitsspeicher (130; 230; 330) mit einem Aufnahmeraum (132; 232; 332) zur Speicherung der pharmazeutischen Flüssigkeit
- einer am Gehäuse vorgesehenen Austragöffnung (112; 212; 312), die den Aufnahmeraum (132; 232; 332) mit einer Umgebung verbindet und der Abgabe der pharmazeutischen Flüssigkeit (136) dient, und
- einer Federeinrichtung (150; 250; 350),
**dadurch gekennzeichnet, dass**
mittels der vorgespannte Federeinrichtung Flüssigkeit im Aufnahmeraum (132; 232; 332) des Flüssigkeitsspeichers (130; 230; 330) druckbeaufschlagt wird und die Federeinrichtung im montierten Zustand der Austragvorrichtung (100; 200; 300) nicht zur Einbringung von Energie zugänglich ist.

2. Austragvorrichtung nach Anspruch 1,
**gekennzeichnet durch**
ein manuell mittels einer Betätigungshandhabe betätigbares Auslassventil (114, 166; 214, 266; 314; 366), welches zwischen dem Aufnahmeraum (132; 232; 332) und der Austragöffnung (112; 212; 312) vorgesehen ist.

3. Austragvorrichtung nach einem der vorstehenden Ansprüchen,
**dadurch gekennzeichnet, dass**
die Austragvorrichtung (100; 200; 300; 400) zur Abgabe der Flüssigkeit in Tropfenform ausgebildet ist.

4. Austragvorrichtung nach einem der vorstehenden Ansprüche,
**dadurch gekennzeichnet, dass**
die Federeinrichtung (150; 250; 350) durch eine Flüssigkeitsspeicherfeder in Form einer elastisch deformierbare Feder (150; 250; 350) gebildet wird, mittels derer der die Flüssigkeit im Aufnahmeraum (132; 232; 332) kraftbeaufschlagt wird, wobei die Flüssigkeitsspeicherfeder (150; 250; 350) insbesondere auf einen den Aufnahmeraum (132; 232; 332) begrenzenden und im Flüssigkeitsspeicher (130; 230; 330) gleitbeweglichen Kolben (140; 240; 340) wirkt.

5. Austragvorrichtung nach einem der vorstehenden Ansprüche, **dadurch gekennzeichnet, dass**
- der Kolben (140; 240, 340) den Aufnahmeraum (132; 232; 332) auf der der Auslassöffnung zugewandten Seite begrenzt und
- die Federeinrichtung (150; 250; 350) derart ausgebildet und/oder angeordnet ist, dass sie den Kolben (140; 240; 340) von der Auslassöffnung (112; 212; 312) weg kraftbeaufschlagt.

6. Austragvorrichtung nach Anspruch 4 oder 5,
**dadurch gekennzeichnet, dass**
- der Flüssigkeitsspeicher (130; 230; 330) als vom Gehäuse (110; 210; 310) getrenntes und mit diesem verbindbares Bauteil ausgebildet ist und
- der Kolben (140; 240; 340) am Gehäuse (110; 210; 310) beweglich gesichert ist.

7. Austragvorrichtung nach einem der Ansprüche 4 bis 6,
**dadurch gekennzeichnet, dass**
der Kolben (140; 240; 340) auf einem Hohlrohr (164; 264; 364) gleitbeweglich geführt ist, welches den Aufnahmeraum (132; 232; 332) des Flüssigkeitsspeichers (130; 230; 330) mit der Auslassöffnung (112; 212; 312) verbindet, wobei das Hohlrohr (164; 264; 364) vorzugsweise fest verbunden oder einstückig mit einem Ventilkörper (166; 266; 366) des Auslassventils (114, 166; 214, 266; 314, 366) ausgebildet ist.

8. Austragvorrichtung nach einem der Ansprüche 4 bis 7,
**dadurch gekennzeichnet, dass**
das Gehäuse (110; 210; 310) und die Relativbeweglich des Kolbens (140; 240; 340) gegenüber dem Gehäuse (110, 210; 310) derart aufeinander abgestimmt sind, dass der Kolben (140; 240; 340) nicht über das der Austragöffnung (112; 212; 312) abgewandte Ende des Gehäuses (110; 210; 310) hinaus gegenüber diesem verlagerbar ist.

9. Austragvorrichtung nach einem der vorstehenden Ansprüche,
**dadurch gekennzeichnet, dass**
ein Ventilkörper (166; 266; 366) des Auslassventils (112, 166; 212, 266; 312, 366) mittels einer Ventilfeder (150; 250; 350) in Richtung eines Schließzustandes kraftbeaufschlagt ist, wobei diese Ventilfeder (150; 250; 350) vorzugsweise mit der Federeinrichtung (150; 250; 350) zur Druckbeaufschlagung der Flüssigkeit im Aufnahmeraum (132; 232; 332) des Flüssigkeitsspeichers (130; 230; 330) identisch ist.

10. Austragvorrichtung nach einem der vorstehenden Ansprüche,
**dadurch gekennzeichnet, dass**
ein von außen zugängliches und bei Inbetriebnahme handhabbares Sicherungsmittel (378, 478) vorgesehen ist, mittels dessen die Federeinrichtung (350, 452) vor Inbetriebnahme im vorgespannten Zustand gehalten wird.

11. Montageverfahren für eine Austragvorrichtung (100; 200; 300) für pharmazeutische Flüssigkeiten,
wobei die Austragvorrichtung (100; 200; 300) umfasst:
- eine erste Baugruppe (102) mit einem Gehäuse (110) mit einer Austrittsöffnung (112) und einem manuell betätigbaren Auslassventil (114, 166),
- eine zweite Baugruppe (104) mit einem Flüssigkeitsspeicher (130) mit einem Aufnahmeraum (132) zur Aufnahme der Flüssigkeit (136) und
- eine Federeinrichtung (150), die im gespannten Zustand die Flüssigkeit (136) im Aufnahmeraum (132) unter Druck setzt,
wobei ausgehend von einem Ausgangszustand, in dem die beiden Baugruppen (102, 104) getrennt vorliegen, folgende Schritte durchgeführt werden:
a. Befüllen des Aufnahmeraums (132) des Flüssigkeitsspeichers (130) mit der pharmazeutischen Flüssigkeit (136) und
b. Verbinden der beiden Baugruppen (102, 104) miteinander, wobei durch das Verbinden die Federeinrichtung (150) gespannt wird und somit der Druck zur Druckbeaufschlagung der Flüssigkeit (136) im Aufnahmeraum (132) aufgebaut wird.

12. Montageverfahren nach Anspruch 11,
**dadurch gekennzeichnet, dass**
die erste Baugruppe (102) einen Kolben (140) und die Federeinrichtung (150) aufweist, wobei nach Befüllen des Aufnahmeraums (132) in Schritt a der Aufnahmeraum (132) zunächst einseitig offen bleibt und erst in Schritt b durch das Einrücken des Kolbens (140) in den Flüssigkeitsspeicher (130) gegenüber der Umgebung verschlossen wird.

## Claims

1. A discharge device (100; 200; 300) for discharging pharmaceutical liquids (136), in particular for ophthalmic use, comprising
- a housing (110; 210; 310),
- a liquid reservoir (130; 230; 330) with a receiving space (132; 232; 332) for storing the pharmaceutical liquid,
- a discharge opening (112; 212; 312) provided on the housing, which connects the receiving space (132; 232; 332) to an environment and which serves for dispensing the pharmaceutical liquid (136), and
- a spring device (150; 250; 350),
**characterized in that**
by means of the pre-tensioned spring device liquid in the receiving space (132; 232; 332) of the liquid reservoir (130; 230; 330) is pressurized and the spring device is not accessible for introduction of energy in the assembled state of the discharge device (100; 200; 300).

2. The discharge device according to claim 1,
**characterized by**
an outlet valve (114, 166; 214, 266; 314, 366) manually actuatable by means of an actuation handle, which valve is provided between the receiving space (132; 232; 332) and the discharge opening (112; 212; 312).

3. The discharge device according to any one of the preceding claims,
**characterized in that**
the discharge device (100; 200; 300; 400) is configured for dispensing liquid in the form of drops.

4. The discharge device according to any one of the preceding claims,
**characterized in that**
the spring device (150; 250; 350) is a liquid reservoir spring in the form of an elastically deformable spring (150; 250; 350), by means of which the liquid in the receiving space (132; 232; 332) is applied with a force, wherein the liquid reservoir spring (150; 250; 350) in particular acts on a piston (140; 240; 340) slidably moveable in the liquid reservoir (130; 230; 330) and limiting the receiving space (132; 232; 332).

5. The discharge device according to any one of the preceding claims,
**characterized in that**
- the piston (140; 240; 340) limits the receiving space (132; 232; 332) on the side facing the outlet opening, and
- the spring device (150; 250; 350) is configured and/or arranged such that it applies a force to the piston (140; 240; 340) away from the outlet opening (112; 212; 312).

6. The discharge device according to claim 4 or 5,
**characterized in that**
- the liquid reservoir (130; 230; 330) is configured as a component separate from the housing (110; 210; 310) and connectable thereto, and
- the piston (140; 240; 340) is movably secured on the housing (110; 210; 310).

7. The discharge device according to any one of the claims 4 to 6,
**characterized in that**
the piston (140; 240; 340) is guided on a hollow tube (164; 264; 364) in a slidable manner, which tube connects the receiving space (132; 232; 332) of the liquid reservoir (130; 230; 330) to the outlet opening (112; 212; 312), wherein the hollow tube (164; 264; 364) is preferably fixedly connected or integral with a valve body (166; 266; 366) of the outlet valve (114, 166; 214, 266; 314, 366).

8. The discharge device according to any one of the claims 4 to 7,
**characterized in that**
the housing (110; 210; 310) and the relative movability of the piston (140; 240; 340) in relation to the housing (100, 210; 310) are matched to one another such that the piston (140; 240; 340) cannot be displaced beyond the end of the housing (110; 210; 310) facing away from the discharge opening (112; 212; 312) in relation to the housing.

9. The discharge device according to any one of the preceding claims,
**characterized in that**
a valve body (166; 266; 366) of the outlet valve (122, 166; 212, 266; 312, 366) is applied with force in the direction of a closed state by means of a valve spring (150; 250; 350), wherein said valve spring (150; 250; 350) is preferably identical to the spring device (150; 250; 350) for pressure application to the liquid in the receiving space (132; 232; 332) of the liquid reservoir (130; 230; 330).

10. The discharge device according to any one of the preceding claims,
**characterized in that**
a securing means (378, 478) is provided which is accessible from the exterior and may be handled during the initial operation, by means of which the spring device (350, 452) is held in a pre-tensioned state prior to an initial operation.

11. An assembly method for a discharge device (100; 200; 300) for pharmaceutical liquids,
wherein the discharge device (100; 200; 300) comprises:
- a first assembly group (102) with a housing (110) having an outlet opening (112) and a manually actuatable outlet valve (114, 166),
- a second assembly group (104) with a liquid reservoir (130) having a receiving space (132) for receiving the liquid (136), and
- a spring device (150), which in the tensioned state pressurizes the liquid (136) in the receiving space (132),
wherein starting from an initial state, where the two assembly groups (102, 104) are present separately, the following steps are performed:
a. filling of the receiving space (132) of the liquid reservoir (130) with the pharmaceutical liquid (136), and
b. connecting the two assembly groups (102, 104) to one another, wherein by means of the connecting, the spring device (150) is tensioned and thus the pressure for pressure application to the liquid (136) in the receiving space (132) is generated.

12. The assembly method according to claim 11,
**characterized in that**
the first assembly group (102) comprises a piston (140) and a spring device (150), wherein after filling the receiving space (132) in step a, the receiving space (132) initially remains open on one side and not earlier than in step b it is closed in relation to the environment by means of inserting the piston (140) into the liquid reservoir (130).

## Revendications

1. Dispositif de distribution (100 ; 200 ; 300) pour distribuer des liquides pharmaceutiques (136), en particulier à des fins ophtalmiques, comprenant
- un boîtier (110 ; 210 ; 310),
- un réservoir de liquide (130 ; 230 ; 330) avec un espace de réception (132 ; 232 ; 332) pour stocker le liquide pharmaceutique,
- une ouverture de distribution (112 ; 212 ; 312) prévue au niveau du boîtier, laquelle relie l'espace de réception (132 ; 232 ; 332) à l'environnement et sert à délivrer le liquide pharmaceutique (136), et
- un dispositif de ressort (150 ; 250 ; 350), **caractérisé en ce que**
du liquide dans l'espace de réception (132 ; 232 ; 332) du réservoir de liquide (130 ; 230 ; 330) est sollicité en pression au moyen du dispositif de ressort précontraint et le dispositif de ressort, dans l'état monté du dispositif de distribution (100 ; 200 ; 300), n'est pas accessible pour l'apport d'énergie.

2. Dispositif de distribution selon la revendication 1,
**caractérisé par**
une soupape de sortie (114, 166 ; 214, 266 ; 314 ; 366) pouvant être actionnée manuellement au moyen d'une manette d'actionnement, laquelle est prévue entre l'espace de réception (132 ; 232 ; 332) et l'ouverture de distribution (112 ; 212 ; 312).

3. Dispositif de distribution selon l'une quelconque des revendications précédentes,
**caractérisé en ce que**
le dispositif de distribution (100 ; 200 ; 300 ; 400) est réalisé pour délivrer le liquide sous forme de gouttes.

4. Dispositif de distribution selon l'une quelconque des revendications précédentes,
**caractérisé en ce que**
le dispositif de ressort (150 ; 250 ; 350) est formé par un ressort de réservoir de liquide sous la forme d'un ressort déformable élastiquement (150 ; 250 ; 350) au moyen duquel le liquide dans l'espace de réception (132 ; 232 ; 332) est sollicité par force, le ressort de réservoir de liquide (150 ; 250 ; 350) agissant notamment sur un piston (140 ; 240 ; 340) limitant l'espace de réception (132 ; 232 ; 332) et pouvant se déplacer par glissement dans le réservoir de liquide (130 ; 230 ; 330).

5. Dispositif de distribution selon l'une quelconque des revendications précédentes,
**caractérisé en ce que**
- le piston (140 ; 240, 340) limite l'espace de réception (132 ; 232 ; 332) du côté tourné vers l'ouverture de sortie et
- le dispositif de ressort (150 ; 250 ; 350) est réalisé et/ou disposé de telle sorte qu'il sollicite par force le piston (140 ; 240 ; 340) à l'écart de l'ouverture de sortie (112 ; 212 ; 312).

6. Dispositif de distribution selon la revendication 4 ou 5,
**caractérisé en ce que**
- le réservoir de liquide (130 ; 230 ; 330) est réalisé sous forme de composant séparé du boîtier (110 ; 210 ; 310) et pouvant être connecté à celui-ci et
- le piston (140 ; 240 ; 340) est fixé de manière déplaçable sur le boîtier (110 ; 210 ; 310).

7. Dispositif de distribution selon l'une quelconque des revendications 4 à 6,
**caractérisé en ce que**
le piston (140 ; 240 ; 340) est guidé de manière déplaçable par glissement sur un tube creux (164 ; 264 ; 364), lequel relie l'espace de réception (132 ; 232 ; 332) du réservoir de liquide (130 ; 230 ; 330) à l'ouverture de sortie (112 ; 212 ; 312), le tube creux (164 ; 264 ; 364) étant de préférence connecté fixement ou étant réalisé d'une seule pièce avec un corps de soupape (166 ; 266 ; 366) de la soupape de sortie (114, 166 ; 214, 266 ; 314, 366).

8. Dispositif de distribution selon l'une quelconque des revendications 4 à 7,
**caractérisé en ce que**
le boîtier (110 ; 210 ; 310) et le piston (140 ; 240 ; 340) déplaçable relativement au boîtier (110, 210 ; 310) sont adaptés l'un à l'autre de telle sorte que le piston (140 ; 240 ; 340) ne puisse pas être déplacé par rapport à celui-ci au-delà de l'extrémité du boîtier (110 ; 210 ; 310) opposée à l'ouverture de distribution (112 ; 212 ; 312).

9. Dispositif de distribution selon l'une quelconque des revendications précédentes,
**caractérisé en ce**
**qu'**un corps de soupape (166 ; 266 ; 366) de la soupape de sortie (112, 166 ; 212, 266 ; 312, 366) est sollicité par force au moyen d'un ressort de soupape (150 ; 250 ; 350) dans la direction d'un état de fermeture, ce ressort de soupape (150 ; 250 ; 350) étant de préférence identique au dispositif de ressort (150 ; 250 ; 350) pour la sollicitation par pression du liquide dans l'espace de réception (132 ; 232 ; 332) du réservoir de liquide (130 ; 230 ; 330).

10. Dispositif de distribution selon l'une quelconque des revendications précédentes,
**caractérisé en ce**
**qu'**il est prévu un moyen de fixation (378, 478) pouvant être manipulé lors de la mise en service et accessible depuis l'extérieur, au moyen duquel le dispositif de ressort (350, 452) est maintenu dans l'état précontraint avant la mise en service.

11. Procédé de montage pour un dispositif de distribution (100 ; 200 ; 300) pour des liquides pharmaceutiques,
le dispositif de distribution (100 ; 200 ; 300) comprenant :
- un premier module (102) avec un boîtier (110) ayant une ouverture de sortie (112) et une soupape de sortie (114, 166) pouvant être actionnée manuellement,
- un deuxième module (104) avec un réservoir de liquide (130) ayant un espace de réception (132) pour recevoir le liquide (136) et
- un dispositif de ressort (150) qui, dans l'état tendu, met sous pression le liquide (136) dans l'espace de réception (132),
les étapes suivantes étant réalisées à partir d'un état de départ dans lequel les deux modules (102, 104) sont séparées :
a. remplissage de l'espace de réception (132) du réservoir de liquide (130) avec le liquide pharmaceutique (136) et
b. connexion des deux modules (102, 104) l'un à l'autre, la connexion tendant le dispositif de ressort (150) et par conséquent augmentant la pression pour la sollicitation en pression du liquide (136) dans l'espace de réception (132).

12. Procédé de montage selon la revendication 11,
**caractérisé en ce que**
le premier module (102) présente un piston (140) et le dispositif de ressort (150), et après le remplissage de l'espace de réception (132) dans l'étape a, l'espace de réception (132) restant d'abord ouvert d'un côté et étant seulement fermé par rapport à l'environnement à l'étape b par l'enfoncement du piston (140) dans le réservoir de liquide (130).
